# EUROPEAN PATENT SPECIFICATION

(11) **EP 3 003 354 B1**
(45) Date of publication and mention of the grant of the patent: **06.02.2019**
(21) Application number: 14731922.2
(22) Date of filing: 30.05.2014
(51) Int. Cl.: A61K 38/18, A61P 9/04

(54) **HUMAN PLGF-2 FOR THE PREVENTION OR TREATMENT OF ATHEROSCLEROTIC HEART FAILURE**
HUMANES PLGF-2 FÜR DIE PRÄVENTION UND BEHANDLUNG VON ATHEROSKLEROTISCHEM HERZVERSAGEN
PLGF-2 HUMAIN POUR LA PRÉVENTION ET LE TRAITEMENT DE LA DÉFAILLANCE CARDIAQUE ATHÉROSCLÉROTIQUE

(30) Priority: 31.05.2013 US 201361829393 P; 16.07.2013 EP 13176638
(43) Date of publication of application: 13.04.2016
(73) Proprietor: CoBioRes NV, 3000 Leuven (BE)
(72) Inventor: JANSSENS, Stefan, B-3001 Heverlee (BE); WU, Ming, B-3001 Heverlee (BE)
(74) Representative: Paemen, Liesbet R.J.
(86) International application number: PCT/EP2014/061259
(87) International publication number: WO 2014/191553

(56) References cited:
- WO-A1-2010/049538
- BINSALAMAH ZIYAD MOHAMMED ET AL: "Intramyocardial sustained delivery of placental growth factor using nanoparticles as a vehicle for delivery in the rat infarct model", INTERNATIONAL JOURNAL OF NANOMEDICINE, 1 October 2011 (2011-10-01), page 2667, XP055076449, ISSN: 1176-9114, DOI: 10.2147/IJN.S25175 cited in the application
- C RONCAL ET AL: "Beneficial effects of prolonged systemic administration of PlGF on late outcome of post-ischaemic myocardial performance", THE JOURNAL OF PATHOLOGY, vol. 216, no. 2, 1 October 2008 (2008-10-01), pages 236-244, XP055076443, ISSN: 0022-3417, DOI: 10.1002/path.2408 cited in the application
- YUKIJI TAKEDA ET AL: "Treatment With Recombinant Placental Growth Factor (PlGF) Enhances Both Angiogenesis and Arteriogenesis and Improves Survival After Myocardial Infarction", CIRCULATION JOURNAL, vol. 73, no. 9, 1 January 2009 (2009-01-01), pages 1674-1682, XP055076444, ISSN: 1346-9843, DOI: 10.1253/circj.CJ-08-1067 cited in the application
- STEPHEN KOLAKOWSKI ET AL: "Placental Growth Factor Provides a Novel Local Angiogenic Therapy for Ischemic Cardiomyopathy", JOURNAL OF CARDIAC SURGERY, vol. 21, no. 6, 1 November 2006 (2006-11-01), pages 559-564, XP055076445, ISSN: 0886-0440, DOI: 10.1111/j.1540-8191.2006.00296.x cited in the application
- HIROTO IWASAKI ET AL: "PlGF Repairs Myocardial Ischemia through Mechanisms of Angiogenesis, Cardioprotection and Recruitment of Myo-Angiogenic Competent Marrow Progenitors", PLOS ONE, vol. 6, no. 9, 28 September 2011 (2011-09-28), page e24872, XP055076459, DOI: 10.1371/journal.pone.0024872 cited in the application
- LIU X ET AL: "Placental growth factor increases regional myocardial blood flow and contractile function in chronic myocardial ischemia", AMERICAN JOURNAL OF PHYSIOLOGY - HEART AND CIRCULATORY PHYSIOLOGY 2013 AMERICAN PHYSIOLOGICAL SOCIETY USA, vol. 304, no. 6, 11 January 2013 (2013-01-11), pages H885-H894, XP009172056, ISSN: 0363-6135 cited in the application
- M WU ET AL: "Systemic recombinant human placental growth factor-2 administration improves myocardial perfusion and contractile function at rest and during stress", EUROPEAN HEART JOURNAL, vol. 34, no. Suppl. 1, 1 August 2013 (2013-08-01), page 415, XP055136433, ISSN: 0195-668X, DOI: 10.1093/eurheartj/eht308.P2272
- IRINA M. JABA ET AL: "NO triggers RGS4 degradation to coordinate angiogenesis and cardiomyocyte growth", JOURNAL OF CLINICAL INVESTIGATION, vol. 123, no. 4, 1 March 2013 (2013-03-01) , pages 1718-1731, XP055076633, ISSN: 0021-9738, DOI: 10.1172/JCI65112 -& IRINA M. JABA ET AL: "NO triggers RGS4 degradation to coordinate angiogenesis and cardiomyocyte growth - Supplemental Data", JOURNAL OF CLINICAL INVESTIGATION, vol. 123, no. 4, 1 April 2013 (2013-04-01) , pages 1-8, XP055155412, ISSN: 0021-9738, DOI: 10.1172/JCI65112
- Anonymous: "Coronary artery disease - Wikipedia, the free encyclopedia", , 24 May 2014 (2014-05-24), pages 1-21, XP055155523, Retrieved from the Internet: URL:http://en.wikipedia.org/w/index.php?ti tle=Coronary_artery_disease&oldid=60998232 1 [retrieved on 2014-11-27]
- LOEHR ET AL: "Heart Failure Incidence and Survival (from the Atherosclerosis Risk in Communities Study)", AMERICAN JOURNAL OF CARDIOLOGY, CAHNERS PUBLISHING CO., NEWTON, MA, US, vol. 101, no. 7, 14 February 2008 (2008-02-14), pages 1016-1022, XP022550838, ISSN: 0002-9149

## Description

### FIELD OF THE INVENTION

The present invention relates to treating and/or preventing heart failure or one or more individual heart failure phenotypes in mammals using placental growth factor 2 (hereinafter referred as PlGF-2).

### BACKGROUND OF THE INVENTION

Despite significant progress in the prevention and treatment of cardiovascular disease, worldwide statistics indicate that the incidence and prevalence of heart failure (HF) continue to rise. In aging societies in industrialized countries the prevalence of HF is 1% between the age of 50 and 59 years, but 10% above the age of 75 years. Moreover, increased survival after myocardial infarction and advances in pharmacological and device therapies for the prevention of sudden cardiac death have markedly enlarged the pool of patients at risk for developing chronic HF.

Previous studies have shown that placental growth factor isoform 2 (PlGF-2) administration can induce angiogenesis and improve myocardial regional and global function in a rodent model of acute myocardial infarction (AMI) (Binsalamah et al. 2011 Int J Nanomed 6, 2667; Roncal et al. 2008 J Pathol 216, 236; Takeda et al. 2009 Circ J 73, 1674). Whether a single dose of placental growth factor isoform 1 (PlGF-1) or a defined sustained delivery period can enhance cardiac global function in a large animal model representative of human disease remains uncertain (Kolakowski et al.2006 J Card Surg 21, 559; Iwasaki et al. 2011 PLos One 6, e24872). It was recently demonstrated that systemic rhPlGF-1 infusion significantly enhances regional blood flow and contractile function of chronic ischemic myocardium without adverse effects. However, the partial improvement in regional contractile functional failed to translate into further recovery of global LV function (Liu et al. 2013 Am J Physiol Heart Circ Physiol 304, H885). Similar knowledge about the effect of P1GF-2 is currently lacking.

Although there are some publications describing particular effects of PlGF1 and PlGF2 on HF parameters in specific models, these fail to provide a straightforward indication of the therapeutic potential of these proteins and moreover do not allow comparison between data obtained for PlGF1 and PlGF2. Indeed, whereas a rat model was used by Binsalamah et al. 2011 (Int J Nanomed 6, 2667), Kolakowski et al. 2006 (J Card Surg 21, 559) and Iwasaki et al. 2011 (PLos One 6, e24872), only Binsalamah et al. (2011) and Kolakowski et al. (2006) intramyocardially administered PlGF2- and PlGF1-protein in saline, respectively. Binsalamah et al. (2011) also intramyocardially administered PlGF2-protein trapped in nanoparticles. Iwasaki et al. (2011) used gene therapeutic intramyocardial administration of PlGF1 (via a PlGF-1 encoding plasmid). Binsalamah et al. (2011) applied echocardiography to determine left ventricular ejection fraction (LVEF%), whereas Kolakowski et al. (2006) used a conductance catheter inserted in the left ventricle using an open chest technique. The results obtained therefore do not allow determination of eventual differences in therapeutic effect between PlGF1 and PlGF2. Roncal et al. 2008 (J Pathol 216, 236) and Takeda et al. 2009 (Circ J 73, 1674) both relied on a mouse model of HF. Whereas Roncal et al. (2008) applied gene therapy (adenovirus vector) to administer PlGF2, Takeda et al. (2009) administered recombinant PlGF1 protein. Again, this difference hinders a reliable comparison of the results obtained and does not allow to capture eventual better therapeutic effects of one PlGF isoform versus the other PlGF isoform. Wu et al. 2013 (Eur Heart J 34 S1, 415) in a porcine model of chronic myocardial ischemia with reduced ejection fraction demonstrated an improved left ventricular ejection fraction and reduced left ventricular end-systolic volume after systemic infusion of P1GF2. The model used by Wu et al. was a non-atherosclerotic model wherein the chronic myocardial ischemia with reduced ejection fraction was induced by implantation of a flow limiting stent.

### SUMMARY OF THE INVENTION

The invention relates to a composition comprising placental growth factor 2 protein (PlGF-2) for use in treating or preventing a heart failure phenotype in an atherosclerotic mammal.

The heart failure phenotype may be defined as any one of, or a combination of: a ventricular dysfunction, an increased end-systolic volume, a reduced ejection fraction, ventricular remodeling, or an increased end-diastolic volume. In particular, the ventricular dysfunction may be defined by an increased end-systolic volume and/or a reduced ejection fraction. Alternatively, the ventricular dysfunction may be defined by an impaired relaxation, an increased ventricular filling pressure and a preserved ejection fraction. In particular, the ventricular remodeling may be defined by an increased end-diastolic volume.

The heart failure phenotype to be treated or prevented may be displayed in the left and/or right ventricle. In a particular embodiment, it is displayed in the left ventricle.

The PlGF-2 protein in the compositions as described hereinabove may be recombinant PlGF-2 protein, such as obtained from a mammalian cell culture. An example of such mammalian cell culture is a Chinese Hamster Ovary (CHO) culture or equipotent cell culture. Such cell culture may be capable of expressing a PlGF-2 protein in a transient or constitutive fashion. The PlGF-2 protein in the compositions as described hereinabove in one embodiment is human PlGF-2 protein, such as defined by SEQ ID NO:1, or polymorphic variants (polymorphs) thereof.

Any of the PlGF-2 protein comprising compositions as described hereinabove is in particular for use by systemic administration. Such systemic administration may be endovascular (e.g. intravenous) or subcutaneous administration.

### FIGURE LEGENDS

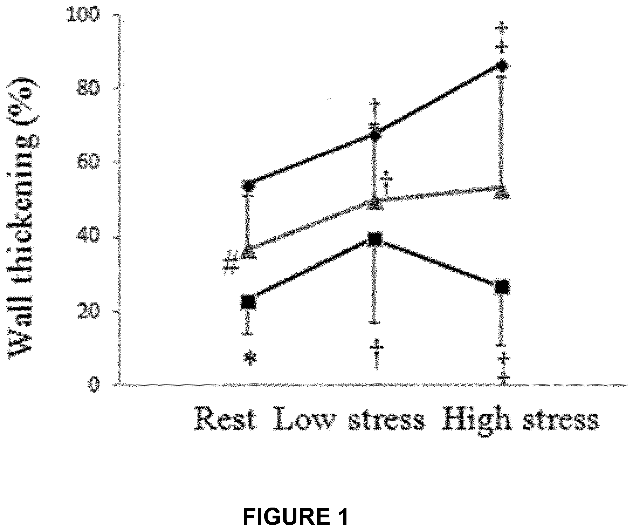

**FIGURE 1****.** RhPLGF2-treatment improves regional myocardial function. Magnetic resonance imaging-derived regional wall-thickening, as indicator of regional myocardial function was better preserved in the ischemic region at rest and during dobutamine-induced stress in rhPLGF2-treated pigs compared to controls. Graph markers: filled square (■) = ischemic, control (phosphate buffered saline), n=12; filled triangle (▲) = ischemic, rhPlGF2-treated, n=12; filled diamond (◆): anterior wall of sham, n=3. Statistic markers: * p<0.05 vs remote and sham; † p<0.05 vs rest; ‡ p<0.05 vs low dose stress; # p<0.05 vs control.

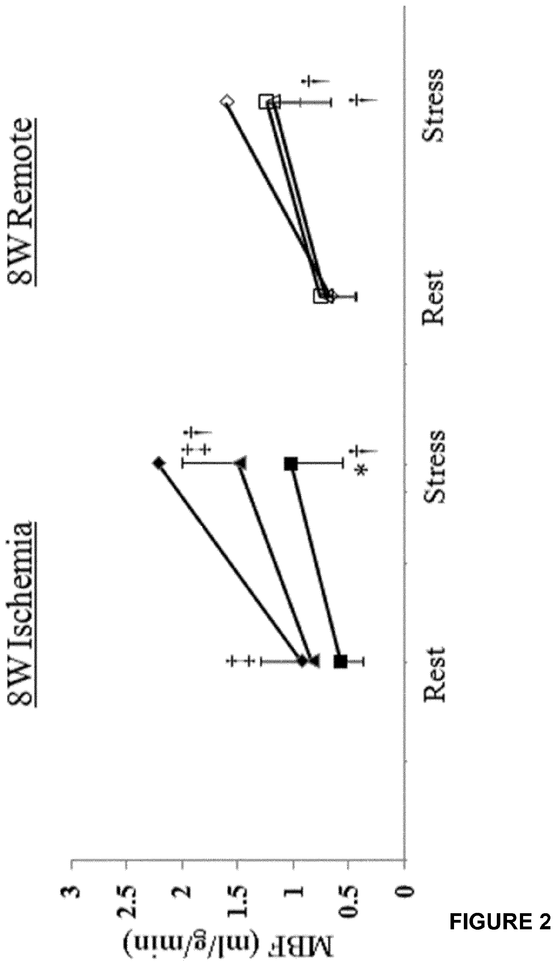

**FIGURE 2****.** Myocardial blood flow (MBF) at rest and during adenosine-induced stress in the ischemic area of rhPLGF2-treated hearts was higher than in controls or in shams (left panel). MBF was higher at stress conditions than at rest, indicating a perfusional reserve. There was no significant difference between the treatment groups in the remote region (right panel). Graph markers for ischemic region (left panel): filled square (■) = control (phosphate buffered saline), n=11; filled triangle (▲) = PlGF-2 treatment, n=11; filled diamond (◆) = sham, n=2. Graph markers for remote region (right panel): square (□) = control (phosphate buffered saline), n=11; empty triangle (Δ) = PlGF-2 treatment, n=11; empty diamond (◊) = sham, n=2. Statistic markers: * p<0.05 vs remote and sham; † p<0.05 vs rest; ‡ p<0.05 vs control.

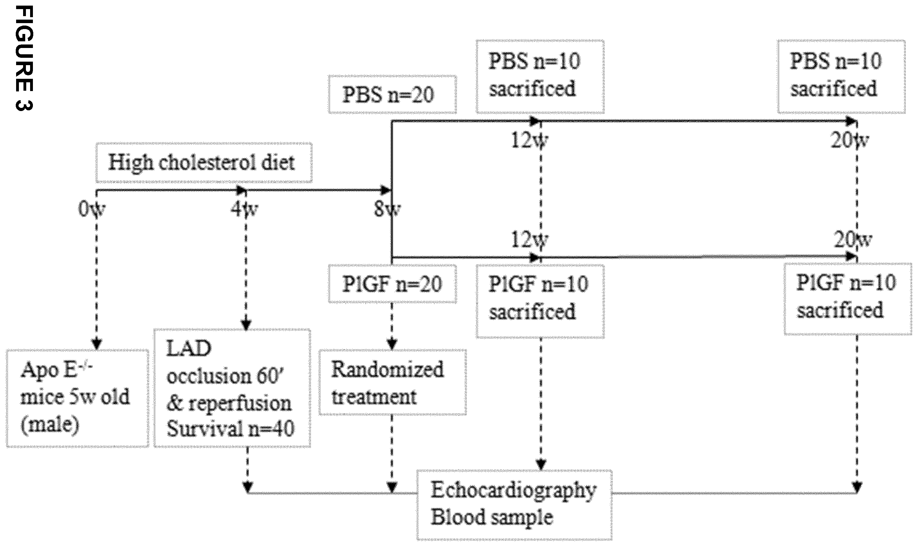

**FIGURE 3****.** Flow-chart depicting the experimental design applied on a murine model of myocardial infarction with reperfusion in atherosclerotic background. LAD = left anterior descending coronary artery; PBS = phosphate buffered saline; PlGF = recombinant human PlGF-2.

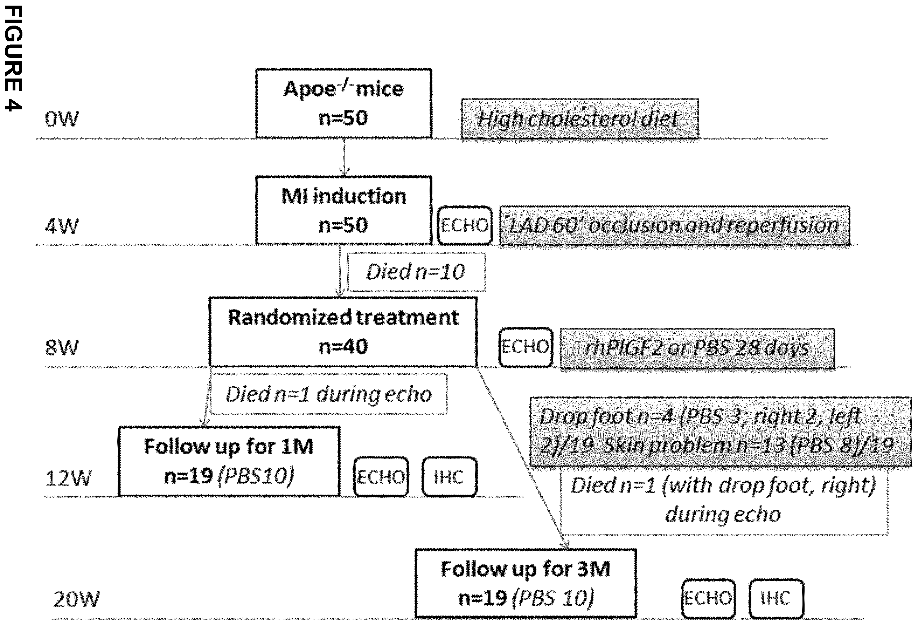

**FIGURE 4****.** Flow-chart of the study protocol on a murine model of myocardial infarction with reperfusion in atherosclerotic background. LAD = left anterior descending coronary artery; PBS = phosphate buffered saline; PlGF = recombinant human PlGF-2; xW = x weeks; ECHO = echocardiography; IHC = immunohistochemistry.

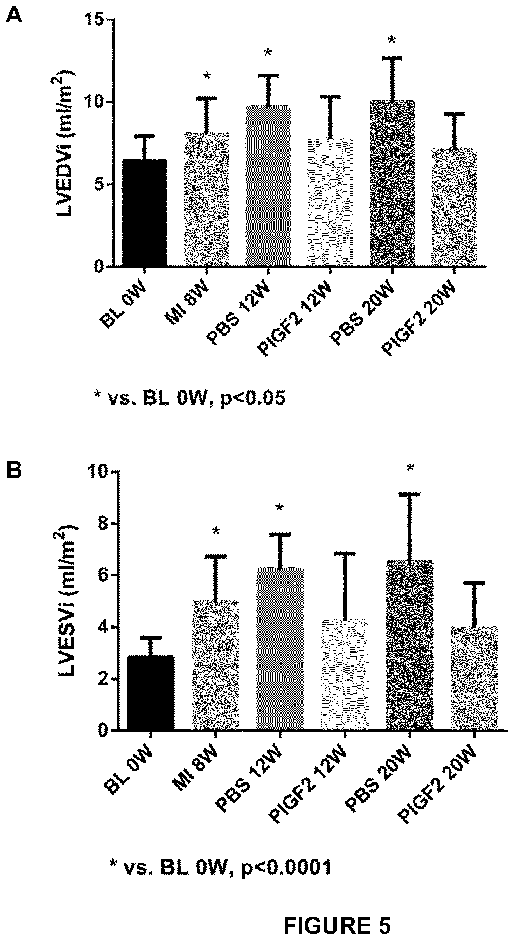

**FIGURE 5****.** Left ventricular (LV) end-systolic and end-diastolic volumes in control- (PBS) and in rhPLGF2-treated groups at time 0W = baseline (BL), at 8 weeks (corresponding to 4 weeks after induction of myocardial infarction and start of treatments), and at 12 and 20 weeks. LV end-diastolic volume indexed to body surface area (Panel A) and LV end-systolic volume indexed to body surface area (Panel B) were significantly increased after MI in the control group, but not after rhPLGF2-treatment. * p<0.05 vs baseline.

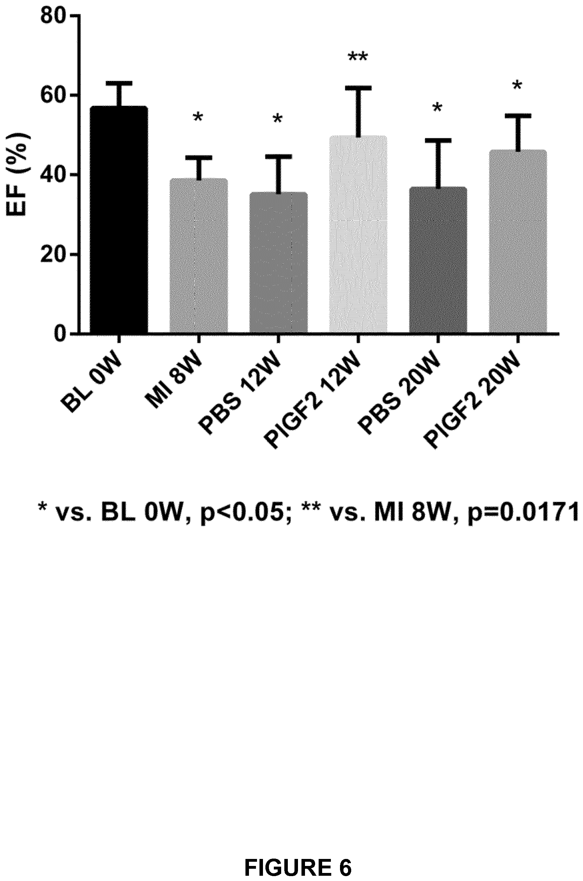

**FIGURE 6****.** Left ventricular (LV) global function in control (PBS) and rhPLGF2-treated groups at time 0 = baseline (BL), at 8 weeks (4 weeks post myocardial infarct induction and corresponding to the start of treatments), and at 12 and 20 weeks. After 12 and 20 weeks, ejection fraction (EF) was significantly higher following rhPLGF2-treatment. * p<0.05 vs baseline; ** p<0.05 vs MI at 8 weeks.

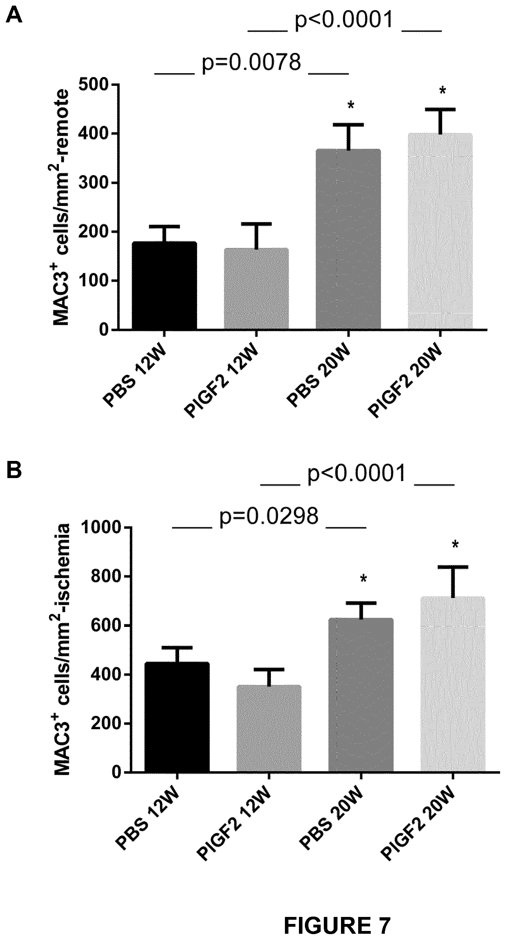

**FIGURE 7****.** Cardiac inflammation in control (PBS) and rhPLGF2-treated mice was evaluated by measuring the number of MAC3-positive cells in the remote heart region (Panel A) and the ischemic areas (Panel B). The number of MAC3-positive cells was higher after 20 weeks compared to 12 weeks in both treatment groups. * p<0.05 vs 12 weeks.

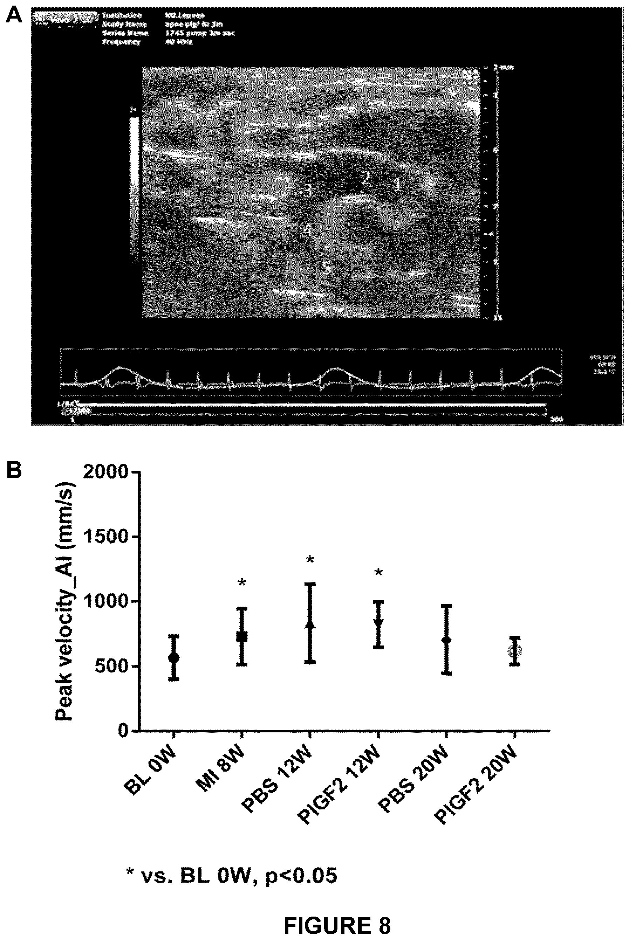

**FIGURE 8****.** Aortic peak blood velocity, measured using Doppler pulse wave ultrasound.
   Panel A: A scanned image of the aorta with the 5 measurement positions: 1 = aortic root; 2 = ascending aorta; 3 = aortic arch next to innominate artery; 4 = aortic arch next to carotid artery; 5 = aortic arch next to subclavian artery.
   Panel B: The aortic arch peak blood velocity (measuring point 3) at baseline (BL; 0 weeks = 0W), 4 weeks after myocardial infarction, corresponding to the start of PBS or rhPlGF-2 administration (MI 8W) and at 12 and 20 weeks. A transient and similar elevation of peak blood velocity occurred at 12 weeks in both groups. * p<0.05 vs baseline.
**FIGURE 9****.** Vascularization in aortic plaques in control (PBS) and rhPLGF2-treated animals was measured and expressed as capillary area related to plaque area (Panel A) and arteriolar area to plaque area (Panel B). Capillary and arteriolar density in the plaques was similar between treatment groups at different time-points.
**FIGURE 10****.** Inflammation response in the atherosclerotic lesions, evaluated as MAC3-positive cell area related to plaque area was comparable between rhPLGF2-treated and control (PBS) groups.

### DETAILED DESCRIPTION OF THE INVENTION

Previous rodent models of acute myocardial infarction caused by permanent ligation of the left anterior descending coronary artery induced massive cardiac ischemia and extensive infarct sizes (Binsalamah et al. 2011 Int J Nanomed 6, 2667; Roncal et al. 2008 J Pathol 216, 236; Takeda et al. 2009 Circ J 73, 1674). The pig model applied in the experiments underlying the current invention uses flow reducing stent technology to mimic the narrowed coronary artery pattern in advanced coronary artery disease patients and is associated with modest myocardial infarction, but induces significant changes in the left ventricular function (contractile dysfunction induced by ischemia) including hampered left ventricular global function indicated by increased end-systolic volume (EDV) and reduced ejection fraction (EF) (Liu et al. 2013 Am J Physiol Heart Circ Physiol 304, H885). The end-diastolic volume (EDV) is not or only marginally increased, this model therefore represents only modest ventricular dilatation. The porcine (i.e. *Sus scrofa,* pig) coronary artery flow-reducing model better represents the clinically relevant ischemic heart disease pathophysiology with moderate heart failure (HF). The herein used mouse model of ischemia-reperfusion in atherosclerotic background (reperfused myocardial infarction with advanced atherosclerotic plaque deposition) allows to study the clinical effect of a compound on cardiac and vascular remodeling, including contractile dysfunction (ischemia-induced; HF phenotypes: increased end-systolic volume and decreased ejection fraction), ventricular remodeling (infarction induced; HF phenotype: increased end-diastolic volume) and pathological vascular flow pattern (atherosclerotic plaque deposition in the main arteries).

Ventricular dysfunction, ventricular remodeling, increased ESV, increased EDV and reduced EF are referred herein further as (some of the) heart failure (HF) phenotypes. Increased ESV and increased EDV are determined as increased volumes when compared to the normal corresponding volumes measured in and derived from a population of healthy subjects. As these volumes are dependent on body weight, normalization to body surface area (BSA) is useful and frequently indexed values (EDVi, ESVi) are used. Reduced EF is determined in comparison to the normal corresponding EF measured in and derived from a population of healthy subjects. EF is the stroke volume (volume determined as EDV - ESV) divided by EDV.

For instance, in the control group of humans assessed by Jorgensen et al. 2007 (Chest 131:1050; see Table 2 therein), left ventricle EDVi is 84± 15 mL/m², left ventricle ESVi is 34±11 mL/m², right ventricle EDVi is 91±15 mL/m² and right ventricle ESVi is 42±11 mL/m². Normal EF in humans is considered to be >50% (ESC Guidelines for diagnosis and treatment of acute and chronic heart failure 2012, Eur Heart J 33:1787), as corroborated by Jorgensen et al. 2007 (see above), noting a left ventricle EF of 60±8% and a right ventricle EF of 54±7%. In ApoE-/- mice as used herein, normal baseline EDV is around 25-35 uL, and normal baseline ESV is around 12-17 uL. As these volumes are dependent on body weight, indexing to body surface area (BSA) is useful. For mice, the following formula can be used to calculate BSA (with BW being body weight in grams): BSA (m²)=(9 x BW^(2/3))/10000. With an average baseline BW = 17.4 ± 1.8 g, the indexed baseline EDV and baseline ESV values are EDVi = 6.4 ± 1.5 (mL/m²) and ESVi = 2.8± 0.8 (mL/m²). For mice, a normal ejection fraction is 55-65%.

Previous studies with PlGF-1 in the pig model were discouraging because no improvement of left ventricular (LV) end-systolic volume (ESV) and LV ejection fraction (EF) at 8 weeks was observed (Liu et al. 2013 Am J Physiol Heart Circ Physiol 304, H885). In work leading to the present invention (Example 1), a significant improvement of both LVESV and LVEF at 8 weeks was observed when administering PlGF-2 under the same conditions as previously used with PlGF-1. In view of the high sequence similarity between PlGF-1 and PlGF-2, this observation is very surprising and unexpected, and adds heart failure as a new indication being treatable by administering PlGF-2. The herein used atherosclerotic mouse model of HF (Example 2) confirms the beneficial effects of PlGF-2 on ventricular function (as in the pig model). In addition, a beneficial effect of PlGF-2 on ventricular remodeling was observed. PlGF-2 moreover proved safe as it did not induce cardiac damage or myocardial inflammation and did not increase plaque instability due to neovascularization or inflammatory cell infiltration. These observations are surprising and unexpected as PlGF is known to be pro-inflammatory and because loss of PlGF delays atherosclerotic lesion development (Roncal et al. 2010, Cardiovasc Res 86:29).

In one aspect, the invention therefore relates to placental growth factor 2 protein (PlGF-2) for use in treating or preventing heart failure or more particularly one or more specific heart failure phenotypes in a mammal as described herein. The invention provides a composition comprising placental growth factor 2 protein (PlGF-2) for use in treating or preventing a heart failure phenotype in a mammal. The heart failure phenotype may be defined as any one of, or a combination of: a ventricular dysfunction, an increased end-systolic volume, a reduced ejection fraction, ventricular remodeling, or an increased end-diastolic volume. In particular, the ventricular dysfunction may be defined by an increased end-systolic volume and/or a reduced ejection fraction. Alternatively the ventricular dysfunction may be defined by an impaired relaxation, an increased ventricular filling pressure and a preserved ejection fraction. In particular, the ventricular remodeling may be defined by an increased end-diastolic volume.

Alternatively, the invention relates to PlGF-2 for use in treating or preventing heart failure in an atherosclerotic mammal. Said heart failure may be accompanied by ventricular dysfunction. It may alternatively or in addition to ventricular dysfunction be accompanied by reduced ventricular ejection fraction or by preserved ventricular ejection fraction.

Alternatively, the invention relates to PlGF-2 for use in improving ventricular ejection fraction of a mammalian heart in an atherosclerotic mammal. Said reduced ventricular ejection fraction may be accompanied by heart failure.

Alternatively, the invention relates to PlGF-2 for use in treating, preventing or improving ventricular dysfuntion in an atherosclerotic mammal. Said ventricular dysfunction may be accompanied by heart failure.

Alternatively, the invention relates to PlGF-2 for use in treating or preventing (hypertrophic) ventricular remodeling of a mammalian heart in an atherosclerotic mammal. Said hypertrophic ventricular remodeling may be accompanied by heart failure.

In heart failure, left ventricle and/or right ventricle may be affected. Therefore, in any of the above, the heart failure or heart failure phenotype to be treated or prevented may be expressed or displayed in the left and/or right ventricle. In a particular embodiment, it is expressed or displayed in the left ventricle.

One of the indications that may lead to myocardial infarction is atherosclerosis, characterized by the deposition of blood-flow limiting plaques in the blood vessels. As demonstrated herein (see Example 2), the compositions as described hereinabove are safe and effective for use in treating or preventing a heart failure phenotype in a mammal suffering from atherosclerosis (an atherosclerotic mammal).

The PlGF-2 protein in the compositions as described hereinabove may be recombinant PlGF-2 protein, such as obtained from a mammalian cell culture. An example of such mammalian cell culture is a Chinese Hamster Ovary (CHO) culture or equipotent cell culture. Such cell culture may be capable of expressing a PlGF-2 protein in a transient or constitutive fashion. The PlGF-2 protein in the compositions as described hereinabove in one embodiment is human PlGF-2 protein, such as defined by SEQ ID NO:1, or polymorphic variants (polymorphs) thereof.

In particular embodiments, the PlGF-2 protein compositions are compositions which consist essentially of the PlGF-2 protein in that the PlGF-2 protein is the only active ingredient. In further particular embodiments, the PlGF-2 protein is the only angiogenic factor in the composition.

Any of the PlGF-2 protein comprising compositions as described hereinabove are in particular
for use by systemic administration. Such systemic administration may be endovascular (e.g. intravenous) or subcutaneous administration.

"Heart failure (HF)" can be defined as an abnormality of cardiac structure and/or function leading to failure of the heart to deliver oxygen at a rate commensurate with the requirements of the metabolizing tissues (ESC Guidelines for diagnosis and treatment of acute and chronic heart failure 2012, Eur Heart J 33:1787). It shows as a complex of clinical signs (e.g. elevated jugular venous pressure, pulmonary crackles and displaced apex beat) and symptoms (e.g. breathlessness, ankle swelling and fatigue) resulting from an abnormality of cardiac function and limiting clinical outcome (i.e. total and cardiovascular death, hospitalization for HF) and quality of life. Heart failure due to ventricular dysfunction encompasses patients with reduced ejection fraction and those with preserved ejection fraction. Ischemic events, including myocardial infarction, can be the underlying cause of heart failure, but other causes include hypertension, valvular heart disease, cardiomyopathy, cigarette smoking, obesity, diabetes, etc. In view of the observations reported herein, heart failure with non-ischemic cause(s) is specifically included as a subgroup of heart failure physiological states in which the cardiac output is insufficient in meeting the needs of the body and/or lungs. A number of HF phenotypes (see above) can be quantitated and serve as measurable parameters, as determined in the pig and mouse models used herein (see Example 1 and 2).

HF with preserved EF, observed in patients with hypertrophic ventricular remodeling with or without important comorbidities including diabetes mellitus, hypertension, chronic obstructive pulmonary disease, renal insufficiency, obesity, has a similarly dismal outcome as HF with reduced EF. The mismatch between perfusion and hypertrophy accounts for the HF phenotype and ventricular dysfunction. Based on this analogy, the scope of this invention includes patients diagnosed with HF with preserved EF. Currently there is no specific therapy to improve clinical outcome in HF with preserved EF.

Another HF phenotype relates to ventricular dysfunction defined by an impaired relaxation, an increased ventricular filling pressure and a preserved EF. In this HF phenotype, the EF is thus preserved, but the ventricles do not relax properly during diastole. If ventricle relaxation is impaired this way, the pressure inside will increase as blood from the next heartbeat tries to enter (increased filling pressure).

Human placental growth factor, hPlGF, was first disclosed by Maglione et al. 1991 (Proc Natl Acad Sci USA 88, 9267) and refers to 3 isoformic variants of a 221 amino acid polypeptide accessible under GenBank accession no. P49763. Subject of the present invention is isoform 2 of PlGF, PlGF-2 (also referred to as PlGF2), which is the isoform comprising a heparin binding site. The full-length reference sequence of hPlGF-2 (i.e. the mature protein lacking the 18-amino acid signal sequence) is included hereafter:

LPAVPPQQWALSAGNGSSEVEVVPFQEVWGRSYCRALERLVDVVSEYPSEVEHM
FSPSCVSLLRCTGCCGDENLHCVPVETANVTMQLLKIRSGDRPSYVELTFSQHVRC
ECRPLREKMKPERRRPKGRGKRRREKQRPTDCHLCGDAVPRR (SEQ ID NO:1).

Sequences of PlGF-2 proteins of other species, in particular mammalian species, can be found by searching in e.g. GenBank, e.g. by running the BLAST program with SEQ ID NO:1. The term "a PlGF-2" in general refers to a PlGF molecule of whatever origin that contains a hPlGF2-like heparin binding domain (HBD). Such hPlGF2-like HBD may be highly homologous (e.g. 95% identical or more) to the HBD of hPlGF2, or it may be less homologous (e.g. 50%, 60%, 70%, 80%, 90%, or less than 95% identical) to the HBD of hPlGF2. The HBD of hPlGF2 is underlined in SEQ ID NO:1 above, and more in particular refers to the insertion of a 21-amino acid sequence RRPKGRGKRRREKQRPTDCHL (SEQ ID NO:2) relative to hPlGF1. The binding of a protein or compound to heparin is easily determinable by means of a heparin binding assay, e.g. by enzyme-linked competitive binding of heparin binding biological molecules (e.g. in 96-well heparin binding plates, BD Biosciences). The term "a PlGF-2" further includes polymorphic variants of a reference PlGF2 sequence for a given genus that may exist in that genus.

Derivatives of PlGF-2 proteins are also intended for use in any of the above described aspects of the invention and the term refers to hybrid molecules which contain at least a PlGF-2 protein portion and an additional portion which differs from that present in the wild-type PlGF-2. Examples of PlGF-2 derivatives include e.g. fusion proteins comprising PlGF-2 and a non-PLGF-related protein (e.g. an albumin or an additional heparin binding domains of protein different from PlGF-2) and fusion proteins comprising PlGF-2 and one or more heparin binding domains of PlGF-2. Derivatives of PlGF-2 further include e.g. addition of a non-protein compound to PlGF-2, e.g. pegylation of PlGF-2. The term PlGF-2 derivative further includes active PlGF-2 muteins. Such muteins may include for instance the mutation of the C-terminal cysteine to another amino acid as described in WO 03/097688. Whatever the nature of the derivation, the resulting PlGF-2 protein derivative should retain its biological activity; it is, however, envisaged that the dose of an active PlGF-2 derivative may be equal, or may need to be higher or lower than the dose of wild-type PlGF-2 in order to obtain the same therapeutic effect. Whereas PlGF-1 binds only to the receptor FLT-1 (and its soluble variant sFLT-1), PlGF-2 additionally binds to neuropilin-1 and -2. Different signaling cascades are triggered by PlGF-1 and PlGF-2 (Kendall et al. 1993, Proc Natl Acad Sci USA 90:10705; Migdal et al. 1998, J Biol Chem 273:22272; Persico et al. 1999, Curr Top Microbiol Immunol 237:31). In view of the different structure, receptor binding pattern and signaling, PlGF-1 is in this context not considered as being a derivative of PlGF-2.

In all of the various aspects of the present invention, the term "mammal" is considered in its common meaning and includes namely humans, equines, felines, canines, porcines, bovines, ovines and the like.

The term "therapeutically effective amount" as used herein preferably means an amount capable of effectuating the envisaged therapeutic effect or effects. It refers to the amount of active ingredient (a PlGF-2 protein or a PlGF-2 derivative) required, in a given delivery method and/or in a given delivery regimen, to produce a beneficial therapeutic effect. A beneficial therapeutic effect includes at least halting or inhibiting further progression of a condition, physiological state or disease. It can, but must not, further includes any level of improvement, amelioration, regression or reduction of, including but not necessarily fully curing, a condition, physiological state or disease. Although the therapeutically effective amount will depend on the delivery method and/or the delivery regimen, it may correspond to an amount of about 2 to 2000 microgram per kg of body weight of the mammal to be treated.

The active ingredient (a PlGF-2 protein or PlGF-2 derivative) is given to the mammal or human patient systemically as protein, e.g. recombinant protein. Recombinant expression of a PlGF-2 protein or PlGF-2 derivative may be in a prokaryotic host (e.g. *Escherichia coli*) although there may be an advantage to produce glycosylated protein such as by expression in eukaryotic cells (e.g. mammalian cell line such as CHO, or insect cells). Such cell lines may be capable to express PlGF-2 or a PlGF-2 derivative either in a transient or in a constitutive fashion.

Systemic delivery (e.g. subcutaneous, or endovascular, e.g. intravenous or intracoronary) has practical advantages over intramyocardial injection. The latter is either performed intraoperatively or via sophisticated catheters. Intramyocardial delivery has the following further limitations: delivery time is unique, repetition requires additional intervention and the injected volume has to be kept minimal to avoid local edema . On the other hand, reaching a sufficient dose of a therapeutic agent via systemic delivery may be hampered by (non-organ specific) side effects. This is for instance the case with the VEGF-A and FGF growth factors, their maximum intravenous or intracoronary doses being limited by their propensity to cause hypotension and edema. Such side effects are, however, not exerted by PlGF (Luttun et al. 2002, Ann NY Acad Sci 979:80). Furthermore, the efficacy and safety of systemic PLGF delivery as demonstrated herein, alleviates the problems faced with previously tested angiogenic therapeutics e.g. VEGF-A and FGF (see Kornowski et al. 2000, Circulation 101:454 for review). Systemic administration of a PlGF-2 protein or PlGF-2 derivative as described above may be obtained via any suitable route (e.g. subcutaneous, intramuscular, intradermal, intravenous, intraarterial, intra-aortal, intracoronary or parenteral administration or by simple catheterization; a further mode of administration is a continuous delivery such as by means of an osmotic pump). Intravenous and/or subcutaneous administration is a preferred route of systemic administration. Not considered to constitute systemic administration of a PlGF-2 protein or PlGF-2 derivative are intramyocardial administration (due to limited systemic spread of administered compound) and administration via gene therapy.

In any of the above, the active ingredient (a PlGF-2 protein or a PlGF-2 derivative) may be included in a formulation further comprising a pharmaceutically acceptable carrier.

The term "pharmaceutically acceptable carrier" means any material or substance with which the active ingredient(s) is formulated in order to facilitate its application or dissemination, for instance by dissolving, dispersing or diffusing the said ingredient, and/or to facilitate its storage, transport or handling without impairing its effectiveness. The pharmaceutically acceptable carrier may be a solid or a liquid, i.e. the compositions of this invention can suitably be used or stored as concentrates, emulsions, solutions, granulates, pellets or powders.

Suitable pharmaceutical carriers for use in the present compositions are well known to those skilled in the art, and there is no particular restriction to their selection within the invention. They may also include additives such as wetting agents, dispersing agents, emulsifying agents, solvents, antibacterial and antifungal agents, isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, coating and/or grinding the active ingredients, in a one-step or multi-step procedure, with the selected carrier material and, where appropriate, the other additives such as surface-active agents, may also be prepared by micronisation, for instance in view of obtaining them in the form of microspheres or nanoparticles for controlled or sustained release of the active ingredients.

Suitable surface-active agents to be used in the pharmaceutical compositions of the present invention are non-ionic, cationic and/or anionic materials having good emulsifying, dispersing and/or wetting properties suitable anionic surfactants include both water-soluble soaps and water-soluble synthetic surface-active agents. Suitable soaps are alkaline or alkaline-earth metal salts, unsubstituted or substituted ammonium salts of higher fatty acids (C₁₀-C₂₂), e.g. the sodium or potassium salts of oleic or stearic acid, or of natural fatty acid mixtures obtainable from e.g. coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanylphosphatidyl-choline, dipalmitoylphoshatidyl choline and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarylesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from 1 to 5 ethyleneglycol units per propyleneglycol unit. Representative examples of non-ionic surfactants are nonylphenol polyethoxyethanol, castor oil polyglycolic ethers, polypropylene/polyethylene oxide adducts, tributylphenoxypolyethoxyethanol, poly ethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, preferably halides, having 4 hydrocarbon radicals optionally substituted with halo-, phenyl-, substituted phenyl- or hydroxyl-groups; for instance quaternary ammonium salts containing as N-substituent at least one C8-C22 alkyl radical (e.g. cetyl, lauryl, palmityl, myristyl, oleyl and the like) and, as further substituents, unsubstituted or halogenated lower alkyl, benzyl and/or hydroxy-lower alkyl radicals. A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbuch", 2nd ed. (Hanser Verlag, Vienna, 1981) and "Encyclopaedia of Surfactants (Chemical Publishing Co., New York, 1981).

Additional ingredients may be included in the formulation of the active ingredient in order to control the duration of action of the active ingredient in the pharmaceutical composition of the invention. Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition may also require protective coatings.

Pharmaceutical forms suitable for injection use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol and the like and mixtures thereof.

### EXAMPLES

### EXAMPLE 1. Effect of PlGF-2 on heart failure in a pig model

### 1.1 PlGF-2 production

A recombinant Chinese hamster ovary (CHO) cell line expressing rhPlGF-2 (recombinant human PlGF-2) was developed using a pEE144hP2 construct in which cDNA sequence of hPlGF-2 was inserted. Glycosylated rhPlGF-2 was generated by sono-perfused high cell density culture in a bioreactor. Glycosylated rhPlGF-2 from CHO cells was purified by different affinity chromatography steps. In a first step cell culture media was concentrated on a hollow fiber with a cut off of 3 kd, so that PlGF-2 (28 kd) did not pass through the membrane. Secondly, after adding 2M ammonium sulfate, the concentrate was further purified on a hydrophobic Octyl Sepharose column. rhPlGF-2 was eluted using 10mM diethanolamine pH 8,5 or in some cases even 40% ethylene glycol in water was required. The eluted fraction was desalted and the buffer was exchanged to PBS using a Sephadex™ G-25 (GE Healthcare). In the last step of affinity chromatography a Heparin Sepharose™ 6 Fast Flow column (GE Healthcare) was used to retain rhPlGF-2 which contains a heparin binding domain. Purified protein was obtained by a step-elution using 1M NaCl in PBS. After pooling the different fractions, purified rhPlGF-2 was stored in PBS after an additional desalting on Sephadex™ G-25 (GE Healthcare) at -20°C.

### 1.2 Study group

This investigation conforms to the Belgian National Institute of Health guidelines for care and use of laboratory animals and the protocol of this double-blind randomized controlled study was approved by the local Ethics Committee on animal research (Ethische Commissie Dierproeven, KU Leuven, Leuven, Belgium).

At day 0, myocardial ischemia was induced using implantation of a flow limiting stent in the left anterior descending coronary artery (LAD) (Liu et al. 2013, Am J Heart Circ Physiol 304:H885). At 4 weeks, after confirmation of chronic myocardial ischemia in comparison with the sham group (Sham), pigs were randomly assigned to blinded rhPlGF-2 (PlGF-2) or PBS (Control) for 10 days. Hemodynamic parameters, magnetic resonance imaging (MRI), and colored microspheres were measured at 4 weeks and 8 weeks. At 8 weeks, pigs were euthanized using an overdose of propofol and saturated potassium chloride. The left ventricle (LV) distal to the stent was sectioned into 5 slices perpendicular to the myocardial long axis from base to apex for microsphere and histological analysis.

Thirty-three crossbred domestic pigs of either gender (*Sus scrofa,* weight 20-25kg, Animalium K.U. Leuven, Leuven, Belgium), were premedicated with 300mg Aspirin (Dispril, Reckitt Benckiser, Brussels, Belgium) and 300mg Clopidogrel (Plavix, Sanofi, Paris, France) 1 day before the intervention. Pigs were sedated with Telazol (tiletamine 4 mg/kg and zolazepam 4 mg/kg) (Zoletil100, Virbac Animal Health, Carros, France) and xylazine (2.5 mg/kg) (Vexylan, CEVA Sante Animale, Brussels, Belgium) and anesthesia was introduced with intravenous propofol (3 mg/kg) (Diprivan, AstraZeneca, Brussels, Belgium) followed by 10mg/kg/h continuous infusion propofol (Diprivan) and remifentanil (18 µg/kg/h) (Ultiva, GSK, Genval, Belgium). Mechanical ventilation with a mixture of air and oxygen (1:1) at a tidal volume of 8-10 ml/kg was adjusted to maintain normocapnia and normoxia, as controlled with arterial blood gas values. Heart rate (HR), rhythm and ST-segment were continuously monitored using electrocardiography. After anticoagulation (heparin 10000 IU) and antiplatelet (acetylsalicylic acid 450 mg) treatment, at day 0, a flow limiting stent was implanted in the left anterior descending coronary artery (LAD) to induce ventrical dysfunction. Aspirin (300 mg) and clopidogrel (75mg) were continued daily during follow-up.

Thirty six pigs were enrolled (3 sham and 33 study group). The flow limiting stent was implanted in the 33 pigs of the study group, of which 7 pigs died of acute complications; 1 pig died at 9 days and another at 11 days due to a large myocardial infarct (25-30% of LV mass). Twenty four pigs were evaluated at 4 weeks, blindly randomized to treatment (12 control and 12 PlGF-2) and reassessed at 8 weeks.

### 1.3 Randomized treatment with PlGF-2 or PBS

Following confirmation of regional myocardial hypoperfusion and dysfunction at rest and during stress using MRI, animals were randomized to a 10-day IV infusion of rhPlGF-2 or PBS via osmotic minipumps. Circulating PlGF levels were quantified using a standard PlGF immunoassay (Quantikine Elisa, R&D systems, Inc), which detects both the human and porcine PlGF-2 isoforms as they share 95% amino acid identity. Plates were coated overnight (4°C) with a monoclonal antibody specifically recognizing PlGF and blocked for 1 hour at room temperature with 1% BSA, washed and incubated with a secondary biotinylated polyclonal goat anti-human PlGF antibody. Bound rhPlGF was detected after incubation with a streptavidin-HRP substrate. Standard dilutions of rhPlGF-2 served as positive control samples.

### 1.4 PlGF levels after 10 days IV infusion

Compared to sham, serum PLGF levels were similar at day 0 before induction of myocardial ischemia and 4 weeks later (10±4 versus 7±0 sham and 14±9 versus 10±2 pg/ml sham, respectively) (3 sham and 24 study group). In contrast, PlGF-2-treated pigs had more than 63-fold higher circulating PlGF levels than control and sham 1 and 2 weeks after minipump implantation (week 5: 799±302 versus 11±5 in control and 13±5 pg/ml in sham, p<0.05; week 6: 867±423 versus 11±4 control and 12±5 pg/ml sham, p<0.05). At 8 weeks, PlGF levels were still more than twofold increased (18±9 versus 8±3 control and 8±1 pg/ml sham, p<0.05) (12 PlGF-2 and 12 control).

### 1.5 Statistical analysis

Statistical analysis was performed using BioStat 2009 statistical software (version 5.8.3.0, AnalystSoft). Data are expressed as mean±SD. ANOVA and a Fisher post-hoc test was used to analyze differences between groups. Student T-test was used to compare differences before and after treatments. When data did not follow a normal distribution, Kruskal-Wallis (KW) non-parametric statistics were reported, and differences between groups identified using Mann-Whitney or Wilcoxon tests.

### 1.6 Left ventricular contractility, measured using invasive pressure-volume technique was significantly improved by PlGF-2 treatment

Neither heart rate nor blood pressure was significantly different compared to sham at 4 weeks. In the ischemic group, the flow limiting stent induced a reduction in systolic function (dP/dtₘₐₓ), and an increase in LV end-diastolic volume. Compared to control, the significant improvement of ejection fraction EF and preload recruitable stroke work (PRSW) was associated with reduced LV end-systolic volume (LVESV) in PlGF-2-treated animals (Table 1).

At 4 weeks, hemodynamic variables were measured with a 5-F Millar pressure-conductance catheter (Millar instruments, Houston, TX, USA) positioned in the left ventricle (LV). Heart rate (HR), LV systolic and end diastolic pressures, and first derivatives of pressure rise and decay (dP/dtₘₐₓ and dP/dtₘᵢₙ) were recorded. At 8 weeks, an instantaneous, intraventricular pressure and conductance measurements were performed. The LV preload was altered with a balloon catheter positioned in the inferior vena cava (IVC). To correct and calibrate the LV volume, cardiac output was determined by thermodilution method using a Swan-Ganz catheter (Edwards Lifesciences LLC, Irvine, California) placed in the pulmonary artery. Blood resistivity was measured with a resistance cuvette (Rho-cuvette) and parallel conductance, attributable to conductance of ventricular wall and connective tissue was evaluated using 10 ml 30% hypertonic saline injections into the IVC. End diastolic volume (EDV), end systolic volume (ESV), ejection fraction (EF), dP/dtₘₐₓ and dP/dtₘᵢₙ were calculated during steady state conditions. The preload-recruitable stroke work (PRSW), a robust, load-independent parameter of ventricular contractility was derived during a transient preload reduction with IVC occlusion. All variables were digitized and processed with PowerLab recording unit and LabChart software(ADInstruments, Oxfordshire, United Kingdom).

Compared to control, the significant improvement of EF and PRSW was associated with reduced LVESV in rhPlGF2-treated animals. The control group had a 2-fold increase in LVESV and a significantly lower EF and PRSW than sham pigs (Table 1).

**Table 1 Left ventricular global function analysis 8 weeks after stenting and 4 weeks after randomized treatment using pressure volume loops**

| | **Control (n=11)** | **PlGF-2 (n=12)** | **Sham (n=3)** |
|---|---|---|---|
| **EDV (ml)** | 152±47 | 122±35 | 94±13 |
| **ESV (ml)** | 105±45 † | 69±31 * | 50±17 |
| **EF (%)** | 41±9 † | 52±11 * | 59±1 |
| **dP/dt** ₘₐₓ **(mmHg/s)** | 1322±438 | 1596±423 | 1997±435 |
| **dP/dt ₘᵢₙ (mmHg/s)** | -1759±507 | -2071±311 | -2402±592 |
| **PRSW (mmHg)** | 41±21 ‡ | 76±24 * | 71±19 |

| | | | |
|---|---|---|---|
| Values are means ± SD. EDV: end-diastolic volume; ESV: end-systolic volume; EF: ejection fraction; PRSW: preload recruitable stroke work. * p<0.05 vs. control; † p<0.05 vs. sham; ‡ p=0.05 vs. sham. | | | |

### 1.7 PLGF2-treatment improves global and regional cardiac function at rest and during dobutamine-stress (cardiac MRI-derived data).

Cardiac MRI was performed on a 3T system (TRIO-Tim, Siemens, Erlangen) at 4 and 8 weeks using electrocardiographic triggering, cardiac-dedicated surface coils and during suspended respiration. Global and regional function was assessed with cine MRI in the vertical and horizontal long and short axes, covering the complete LV using 6-mm thick slices, at rest and during dobutamine-induced stress. Dobutamine (Dobutrex, Merck, Overijse, Belgium) was intravenously infused at 5µg/kg/min and then titrated up to reach a target heart rate of 80% above baseline. Regional perfusion at rest and during stress (adenosine 180µg/kg/min) was evaluated using dual-bolus first-pass imaging. Myocardial viability was evaluated using late contrast-enhanced (LE) MRI.

A complete description of the MRI sequences has been reported before (Wu et al. 2011, Cardiovasc_Res 89:166). All MRI studies were analyzed using dedicated software with investigators blinded to the treatment allocation. Myocardial regional perfusion, function and LE were evaluated in identical anatomical regions. On these slices the delineations of LV epicardial and endocardial borders were divided into 6 equiangular segments. For assessment of global LV function, endocardial and epicardial borders were traced in end-diastolic and end-systolic short-axis slices. We calculated LV EDV and ESV, stroke volume (SV) and EF as an index for global function. LV mass and all volumes were reported indexed to the body surface area (Schmidt-Nielsen 1984, "Scaling: Why is Animal Size so Important?", New York: Cambridge University Press). Myocardial wall thickening was measured as an index of regional function in remote and ischemic region. Myocardial blood flow (MBF) was calculated as a parameter of regional perfusion in remote and ischemic area. Since hyperemic perfusion can accurately define the ischemic region in chronic ischemia, the ischemic region was defined using the segmental thresholds (mean-t0.95, n-1*sd*sqrt((n+1)/n)) obtained in the sham group during stress perfusion at 4 weeks. For calculation of myocardial infarct size endocardial and epicardial borders and LE regions were contoured. Myocardial infarct size (IS) was calculated as total LE volume normalized to LV myocardial volume.

After PlGF-2 infusion, EF significantly improved from 4 to 8 weeks compared to PBS treatment. This improvement is associated with a reduction in end-diastolic volume index ESVi (Table 2A). The flow limiting stent implantation induced limited infarction at 4 weeks (infarct size IS 4±6%, ranging 0-15%). In both groups the infarct size (IS) was comparable. Of note, IS was unchanged from 4 to 8 weeks (Table 2A). For purposes of easy comparison, the relevant original data reported for PlGF-1 in the same model as used herein for assessing PlGF-2 (Table 4 in Liu et al. 2013 Am J Physiol Heart Circ Physiol 304, H885) are included hereafter in Table 2B. It should be noted, however, that the published values reflect means ± standard error (SE) whilst the values given in Table 2B reflect means ± standard deviation (SD). Contrary to the effects of PlGF-2, PlGF-1 does not improve EF, nor does it reduce ESVi.

**Table 2A. Left ventricular global function and structure analysis using MRI; PlGF-2 study.**

| | **Control (n=12)** | | **PLGF-2 (n=12)** | | **Sham (n=7)** | |
|---|---|---|---|---|---|---|
| | Absolute value | Change vs. 4 w | Absolute value | Change vs. 4 w | Absolute value | Change vs. 4 w |
| **BW (kg)** | 64±5 | 16±5 | 61±5 | 19±5 | 63±12 | 18±6 |
| **LVMi (g/m²)** | 56±7 | -6±4 | 60±9 | -2±7 | 67±18 | 0±6 |
| **EDVi (ml/m²)** | 111±35 | -7±16 | 105±32 | -15±21 | 91±15 | -6±13 |
| **ESVi (ml/m²)** | 64±32 | -1±11 | 53±29 | -13±9† | 40±6 | -5±6 |
| **SVi (ml/m²)** | 47±10 | -6±6 | 51±12 | -2±15 | 51±10 | -1±8 |
| **EF (%)** | 44±11 * | - 2±2 | 51±11 | 5±6† | 56±3 | 2±2 |
| **IS (% of LV)** | 4±6 | 0±2 | 4±6 | 0±1 | | |

| | | | | | | |
|---|---|---|---|---|---|---|
| Values are means ± SD. EDVi: end-diastolic volume index; ESVi: end-systolic volume index; SVi: stroke volume index; EF: ejection fraction; LVMi: left ventricular mass index; IS: infarct size. Volumetric parameters were indexed to body surface area. * p=0.01 vs. sham 8w; † p<0.001 vs. control. | | | | | | |

**Table 2B. MRI analysis of global LV function at 8 weeks after stent implantation; PlGF-1 study.**

| | Control (n=9) (mL/min/ g) | PLGF-1 (n=8) (mL/min/ g) | Sham (n=4) (mL/min/ g) |
|---|---|---|---|
| **BW (kg)** | 65±6 | 63±8 | 64±14 |
| **LVMi (g/m²)** | 81±9 | 80±20 | 74±12 |
| **EDVi (ml/m²)** | 76±18 | 81±25 | 80±16 |
| **ESVi (ml/m²)** | 45±12* | 49±20* | 35±4 |
| **SVi (ml/m²)** | 31±6* | 32±6* | 44±14 |
| **EF (%)** | 41±6* | 41±8* | 54±8 |

| | | | |
|---|---|---|---|
| Values are expressed as **means ± SD.** Volumetric parameters were indexed to body surface area (BSA) using Meeh's formula as follows: BSA (in m²) = *k* x [body weight (in kg)]^{2/3} x 10⁻², where *k* = 9.0. BW: body weight; EDVi: end-diastolic volume index; ESVi: end-systolic volume; LVMi: left ventricular mass index; SVi: stroke volume index; EF: ejection fraction. *p<0.05 vs sham. | | | |

At 8 weeks, in sham animals, wall thickening at rest was comparable in the LAD perfusion territory and remote areas (54±1% versus 55±1%) and showed similar progressive responses to incremental dobutamine stress. In the ischemic areas rhPLGF-2-treated pigs wall thickening at rest was significantly and selectively increased compared to control (37±15% versus 23±9%, p=0.02), whereas PBS had no effect. Contrary to the biphasic response to stress in the control group, after rhPLGF-2 treatment wall thickening increased during low dose stress without further increments during high-dose stress showing a plateau-phase in the ischemic region. In the remote area, wall thickening was comparable at rest and during all phases of stress among the rhPLGF-2, control or sham groups (Figure 1).

Four weeks after ischemic stent implantation both at rest and after hyperemic stimulation myocardial blood flow (MBF) in the ischemic region was significantly reduced compared to sham (rest: 0.63±0.18 versus 0.87±0.06 ml/min/g, p=0.03 and stress: 0.94±0.33 versus 1.74±0.01 ml/min/g, p=0.0004), although there was still residual perfusion reserve. At 8 weeks, in the rhPLGF-2-treated group MBF both at rest (0.83±0.32 versus 0.58±0.21 ml/min/g, p=0.03) and at hyperemic perfusion (1.50±0.50 versus 1.02±0.46 ml/min/g, p=0.03) increased significantly, but not in controls. In the remote region, MBF at rest and after hyperemic perfusion was comparable among rhPLGF-2-treated, control and sham animals (Figure 2).

### 1.8 Microsphere measurements

After obtaining hemodynamic data, a 6-F pigtail catheter was inserted in the LV and 6 million colored microspheres (15-µm diameter; Triton Technologies, Inc) were injected to measure regional myocardial blood flow (MBF). Absolute blood flow was quantified by comparing microsphere concentrations in different myocardial regions to those measured in a reference blood sample. Reference blood sample was withdrawn from the abdominal aorta at a constant rate and started 20 seconds before the microsphere injection. Two reference tissue samples were obtained from right and left kidney to test homogeneity of the microsphere distribution. Myocardial samples from the ischemic and remote area (inferior wall) and reference blood and kidney samples were analyzed using a luminescence spectrophotometer (Agilent 8453E UV-visible spectroscopy system). Congruent with MRI measurements, MBF determined using colored microspheres increased significantly from 4 weeks to 8 weeks in the rhPLGF2-treated animals, but not in controls (Table 3).

**Table 3. Absolute values of regional myocardial blood flow in the ischemic area.**

| | **MRI analysis** | | **Microsphere analysis** | |
|---|---|---|---|---|
| | ***4W*** | ***8W*** | ***4W*** | ***8W*** |
| **Control (ml/min/g)** | 0.64±0.22 | 0.58±0.21 | 0.58±0.32 | 0.66±0.47 |
| **rhPlGF2 (ml/min/g)** | 0.63±0.14 | 0.83±0.32 * | 0.62±0.21 | 0.95±0.55 * |

| | | | | |
|---|---|---|---|---|
| Values are means ± SD. * p<0.05 vs. regional myocardial blood flow at 4 weeks. | | | | |

### 1.9 Biomarker analysis, neovascularization measurement

Cardiac necrosis markers (TnI, CK, CKMB, CKMB relative index and LDH) and liver function (AST and ALT) tests were analyzed at day 0 before stent implantation, at 4 weeks before treatment, 5, 6 and 8 week follow-up. These values were comparable in rhPLGF2, control and sham groups.

### 1.10 PlGF-2 induces neovascularization of the ischemic myocardium

Neovascularization was evaluated on 5-µm paraffin-embedded sections of the ischemic and remote zones. All images were analyzed in 10 randomly selected high-power fields (HPF) from each zone. Lectin and smooth muscle cell (SM-actin) markers were used to label capillaries and small muscularized vessels, respectively. Density of capillaries and muscularized arterioles was assessed by counting the number of lectin- and SM-actin positive vessels and expressed relative to the tissue area.

In rhPLGF2-treated pigs, lectin-stained capillary and smooth muscle cell actin-detectable arteriole densities were significantly higher in ischemic areas than in controls (1691±509 versus 1101±445 number of capillaries/mm² control, p<0.05; 69±29 versus 39±13 number of small muscularized vessels/mm² control, p<0.05).

### 1.11 PlGF-2 safety analysis after 10 day infusion

Cardiac necrosis markers and liver function tests were comparable among 3 groups and within the physiological ranges.

### 1.12 Conclusion

In a clinically representative model (i.e. *Sus scrofa*) of HF with reduced ejection fraction (EF) the effect of recombinant human PlGF-2 to placebo on left ventricular dysfunction were compared. It was demonstrated using invasive hemodynamic measurements that global cardiac function, represented by EF improvement from 41±9% to 52±11%. In addition, using comprehensive magnetic resonance imaging it was confirmed that PlGF-2-mediated improved EF from 44±11% to 51±11%, respectively. The combined data indicate that the prevention of cardiac dysfunction following PlGF-2-treatment is attributable to a lesser increase in end-systolic volume (ESV) over time (ΔESVindex: -13±9 ml/m² in PlGF-2 group versus -1±11 ml/m² in controls). These observed changes in EF (ΔEF) were +5±6% in PlGF-2-treated animals versus -2±2% in controls. The magnitude of ΔEF was similar to changes observed during previous landmark HF studies using beta-blockers and ACE-inhibitors with conferred significant mortality benefit. The results of these clinical studies have led to the integration of the latter agents in standard-of-care treatment protocols for HF. Surprisingly, the clinical effects of PlGF-2 on HF as demonstrated herein (improvement of HF phenotypes ESV and EF) are superior to those of PlGF-1 used in the same preclinical HF model.

### EXAMPLE 2. PlGF-2 therapy for myocardial ischemia in an atherosclerotic mouse model

### 2.1 Introduction

In the previous Example, it was demonstrate that systemic administration of recombinant human placental growth factor 2 (rhPlGF-2) in pigs with chronic myocardial ischemia significantly enhances regional myocardial blood flow and left ventricular contractile function at rest and during stress. Moreover, sustained delivery of rhPlGF-2 also resulted in a prominent recovery of global cardiac function without adverse effects. However, the non-atherosclerotic porcine model we studied precludes direct extrapolation to patients with atherosclerosis, in whom angiogenic growth factors could induce intimal hyperplasia and progression of coronary atherosclerotic lesions (Celletti et al. 2001, Nat Med 7:425). A previous experimental study in atherosclerotic rabbits reported that local adenoviral PlGF-2 delivery significantly increases intimal thickening and macrophage accumulation in the collared carotid arteries. Also, the size and macrophage content of early atherosclerotic lesions were reduced in mice deficient in both apolipoprotein (apo) E and PlGF compared with apo E deficient (Apo E^{-/-}) mice (Khurana et al. 2005, Circulation 111:2828). In another study, after 5 weeks delivery of anti-PlGF antibody, inflammatory cell infiltration in atherosclerotic plaques and plaque size were reduced in an early stage of mild atherosclerosis (Roncal et al. 2010, Cardiovasc Res 86:29). It remains unknown whether increased PlGF level reflect the severity of the myocardial ischemia or contribute to lesion progression. The inflammatory response of myocardial and vascular tissues following PlGF-2 infusion in a murine model of advanced atherosclerosis and chronic myocardial infarction/ischemia were therefore investigated. The effect of sustained systemic administration of PlGF-2 on different parameters was evaluated: myocardial neovascularization, cardiac perfusion and function, cardiac remodeling, size of atherosclerotic plaques, macrophage accumulation and activation of the plaques after 5 months.

### 2.2 Materials and methods

### 2.2.1 Study design

This investigation conforms to the Belgium National Institute of Health guidelines for care and use of laboratory animals and the protocol of this double-blind randomized controlled study was approved by the local Ethics Committee on animal research (Ethische Commissie Dierproeven, KU Leuven, Leuven, Belgium).

Five week old male Apo E^{-/-} mice (B6.129P2-APOE/J, n=50, body weight 17.4±1.8 g) bought from Charles River Laboratories were used in the study. All mice were ad libitum fed with high cholesterol (1.25%) diet (TD.88137, Teklad, Harlan Laboratory) during the whole experiment. After 4 weeks, atherosclerotic lesions in aorta, cardiac morphology, global and regional function were assessed using ultrasound imaging. Then left descending coronary artery (LAD) occlusion (60 min) followed by reperfusion was performed to induce myocardial infarction (MI). At 8 weeks, after evaluation of chronic myocardial infarction/ischemia and aortic atherosclerosis, mice were randomly assigned to blinded PlGF-2 (450µg/kg/day) (recombinant human PlGF-2 derived from CHO cells; Cat. No. 6837-PL, R&D systems, UK) or phosphate buffered saline (PBS) treatment using osmotic mini pumps (Alzet model 2004, Charles River Laboratories, France) for 28 days. At 12 weeks, half of the mice from each group underwent ultrasound imaging and post mortem pathological analyses. The remaining mice were followed up to 20 weeks and similar analyses were repeated (a flow-chart of the experiment is given in Figure 3). Blood samples were collected for determination of total cholesterol, PlGF level, cardiac necrosis and inflammation markers. After euthanasia, the heart, aortic artery, spleen and tibia were harvested for histological analysis.

### 2.2.2 Induction of myocardial infarction

After anesthesia with Nembutal (60 mg/kg), mice were placed in a supine position on a heating pad (37°C) to prevent hypothermia during anesthesia. Mechanical ventilation was at a tidal volume of 250 µl and rate of 150 per minute to maintain normocapnia and normoxia.

The heart was exposed via a thoracotomy in the intercostal space between the third and fourth rib. A 7-0 suture was placed to ligate the LAD for 60 min. To release the ligature, the silk snare was released, and reperfusion was confirmed by visual inspection. Then the chest was closed. After surgery mice were allowed to recover from anesthesia, and the endotracheal tube was removed once spontaneous breathing resumed. Post-operative subcutaneous Buprenorphine (0.05-0.2 mg/kg) analgesia was applied.

### 2.2.3 Randomized treatment with PlGF or PBS

Four weeks after myocardial infarction, animals were randomized to a 28-day subcutaneous infusion of PlGF-2 (recombinant human PlGF-2 derived from CHO cells; Cat. No. 6837-PL, R&D systems, UK) or PBS via osmotic minipumps. Circulating PlGF levels were quantified using a standard PlGF immunoassay (Quantikine Elisa, R&D systems, Inc), which detects both the human and murine PlGF-2 isoforms. Plates were coated overnight (4°C) with a monoclonal antibody specifically recognizing PlGF and blocked for 1 hour at room temperature with 1% BSA, washed and incubated with a secondary biotinylated polyclonal goat anti-human PlGF antibody. Bound PlGF was detected after incubation with a streptavidin-HRP substrate. Standard dilutions of PlGF-2 served as positive control samples.

### 2.2.4 Ultrasound imaging measurements

Transthoracic ultrasound imaging was performed under light anesthesia (1-2% isoflurane in oxygen). Animals were placed supine on an electrical heating pad at 37°C with continual ECG and respiration monitoring. Cardiac and Vascular images were acquired with 18-38 and 32-56 MHz high frequency linear-array transducers (MS400 and MS550S, respectively) with a digital ultrasound system (Vevo 2100 Imaging System, VisualSonics, Toronto, Canada).

A 2D echocardiographic study was performed in parasternal long-axis (Bhan et al. 2014, Am J Physiol Heart Circ Physiol 306, H1371). For optimized image quality, image depth, width and gain settings were adapted accordingly. The average depth was 14 mm and all frame rate above 200 frames per second. Images were digitally stored in cine loops consisting of 300 frames.

The blood flow velocity in the ascending aortic artery and the aortic arch were characterized at different anatomical points using pulse wave Doppler images. Doppler velocity measurement was performed with the smallest possible angle of incidence between the Doppler beam and the assumed blood flow direction in the middle of the targeted position.

All ultrasound imaging studies were analyzed using the Vevo 2100 (1.5.0) software with investigators blinded to treatment allocation.

Left ventricular (LV) volume and function were evaluated using speckle tracking analysis. Suitable B-mode loops were selected based on adequate visualization of the endocardial border and absence of image artifacts. Three consecutive cardiac cycles were selected for analysis based on image quality. Semiautomated tracing of the endocardial and epicardial borders were performed and verified over all 3 cardiac cycles and then corrected as needed to achieve good quality tracking throughout each cine loop. Tracked images were then processed in a frame-by-frame manner. LV end-systolic volume (LVESV) and LV end-diastolic volume (LVEDV) were calculated using a method of disks technique from the tracing. LVEF and LV mass were derived from the volume. LV mass and all volumes were reported indexed to the body surface area (Schmidt-Nielsen 1984, see supra). Peak systolic longitudinal and radial strain measures were obtained during regional speckle-tracking analysis (Thibault et al. 2011, Circ Cardiovasc Imaging 4:550; Bauer et al. 2011, Circ Res 108:908). Each long-axis view of the LV myocardium was divided into 6 standard anatomic segments throughout the cardiac cycle. On the images recorded 1 month after MI, ischemic region was defined on mid-anterior, apical-anterior, and apical-inferior wall segments and taking into account the wall motion abnormality. The basal-inferior and mid-inferior wall segments were designated as the remote region. Regional strain values were obtained by averaging these same measurements across infarct and remote segments, respectively.

The blood flow peak velocity as an index of arterial stiffness was extracted and averaged for 3 consecutive cardiac cycles.

### 2.2.5 Total cholesterol, C reactive protein (CRP) and cardiac troponin I (cTnI) measurement

Total cholesterol level was analyzed at 0 week, 1 month and 3 months after treatment. High sensitive CRP as an indicator of inflammation was measured at 0 week and at 2 endpoints. Cardiac TnI was analyzed at 0 week 3 hours after ischemic reperfusion and 2 endpoints.

### 2.2.6 Histopathological analysis

The heart was arrested in diastole after injection of 200µl oversaturated KCl via vena cava. The tissues were perfused with heparinized saline and fixed with zinc formalin fixative (Z2902, Sigma). The heart, aortic artery, spleen and tibia were harvested. The wet weights of heart and spleen and lengths of tibia were measured. The hearts and aortic arches were stored in fixative overnight, then in 70% ethanol and embedded into paraffin using standard procedure and sectioned to 5 µm thick slices. The hearts were sliced transversely from apex to base part of the LV and the aortic arches longitudinally.

On the cardiac slices, fibrosis, angiogenesis and inflammation were assessed. Fibrosis and infarct size (IS) were quantified on Sirius red stained slices. The minimum number of sections per heart required for reliable measurement of infarct size is 1/3 of the total 20 slices (Takagawa et al. 2007, J Appl Physiol 102:2104). We analyzed 7 slices at regular interval to cover the whole LV. The red-stained collagen was segmented (isolated) using thresholding. Infarct area was derived from the thresholded area and the LV area was traced manually. IS was calculated by dividing the sum of infarct areas from all sections by the sum of LV areas from all sections and multiplied by 100. Neovascularization and inflammation was evaluated on the ischemic and remote zones. All images were analyzed in 8 randomly selected high-power fields (HPF) from each zone. Capillary and arteriolar densities were assessed by the number of lectin and SM-actin positive vessels over the tissue area. The inflammation response was evaluated using MAC3 immunohistochemical staining. Inflammation was expressed as the number of MAC3- positive cells over the tissue area.

On the aortic arch slices, the atherosclerotic plaques at the minor curvature of the arch were analyzed for plaque size, composition, vascularization and inflammation. Verhoeff van Gieson exclusively stains the elastic laminae. Plaque area was measured as the difference between lumen area and the area delimited by the internal elastic lamina, and the results were normalized to total vessel area to eliminate variations due to vessel size. The plaque collagen and calcification area stained positive for Sirius red and alizarin red. Lectin and SM-actin were used to label capillaries and small muscularized vessels in the plaque. MAC3 staining was used to quantify the inflammation response. Images were acquired and analyzed using thresholding to determine the total positive staining for each method. These results were then normalized to total vessel area.

### 2.2.7 Statistical analysis

Statistical analysis was performed using GraphPad Prism 6 software. Data are expressed as mean±SD. ANOVA and a post-hoc test was used to evaluate the evolution of the model from 0 week, 8 week to 1 month and 3 month control and from 0 week, 8 week to 1 month and 3 month treatment, and to analyze differences between groups. Student T-test was used to compare differences before and after treatments. When data did not follow a normal distribution, Kruskal-Wallis (KW) non-parametric statistics were reported, and differences between groups identified using Mann-Whitney or Wilcoxon tests.

### 2.3 Results

### 2.3.1 Study group

Fifty Apo E^{-/-} male mice were enrolled and fed ad libitum with high cholesterol diet. Four weeks later, we performed 60 min LAD occlusion and reperfusion in all mice, of which 10 mice died of acute ischemic complications. Forty mice were evaluated at 8 weeks, blindly randomized to treatment (20 PBS and 20 PlGF). Half of them (10 PBS and 10 PlGF) were reassessed at 12 weeks and the other half at 20 weeks. Two mice from PlGF group (one at 12 week and the other at 20 weeks) died during ultrasound examination. After 20 weeks follow up, 4 out of 19 mice were found upper limb drop foot (2 right and 2 left side, 3 from PBS group). Thirteen mice had skin problems (8 from PBS group). The study flow chart is depicted in Figure 4.

### 2.3.2 PlGF levels after 28 days subcutaneous infusion

Plasma PlGF levels were similar at week 0 and week 8, that is 1 month after induction of MI (0±0 vs. 0.4±2.1 pg/ml at week 8). In PBS group, PlGF values from week 9 till week 20 were comparable to week 0 and week 8. In contrast, PlGF-treated mice had 1653-, 92-, and 23-fold higher circulating PlGF levels than PBS at 1, 2 and 3 weeks after minipump implantation (week 9: 5781±3710 versus 3.5±8.6 pg/ml PBS, p<0.0001; week 10: 168.8±547.1 versus 1.8±4.6 pg/ml PBS, p=0.0009; week 11: 42.0±78.0 versus 1.8±5.4 pg/ml PBS, p=0.019). At 12 and 20 weeks, PlGF levels returned to the levels of week 0 and week 8.

### 2.3.3 PlGF-2 has no significant effect on the cardiovascular risk factors

The PlGF2 treatment did not change significantly the cardiovascular risk factors, including the body weight, total cholesterol level, high sensitive CRP level, heart weight and normalized heart weight (heart weight over body weight and heart weight over tibia length).

### 2.3.4 PlGF-2 improves cardiac function and prevents LV remodeling: LV volume, cardiac global and regional function analysis using echocardiographic speckle tracking imaging.

Heart rate was significantly increased after myocardial infarction and raised further until 12 weeks and remained elevated till 20 weeks. The LAD ischemic reperfusion induced a reduction in EF, and an increase in LVEDVi and LVESVi. Compared to PBS, the significant improvement of EF was associated with reduced LVEDVi and LVESVi in PlGF-2-treated animals at 12 weeks. PlGF-2 infusion prevented additional LVEDVi, LVESVi elevation and EF deterioration at 20 weeks. This confirms the beneficial effect of PlGF-2 on global LV structure (Figure 5) and function (Figure 6).

Four weeks after MI, both radial and longitudinal strain in the ischemic region decreased significantly compared to week 0. After PlGF-2-treatment, the regional radial and longitudinal strain transiently improved at 12 weeks.

### 2.3.5 PlGF-2 induces neovascularization in ischemic myocardium

In PlGF-2-treated mice, lectin-stained capillary density was significantly higher after 1 month in the ischemic areas than in controls (2144±478 versus 2813±212 number of capillaries/mm², p<0.05 versus control). SM actin-stained arteriolar density became significantly higher in PlGF-2-treated group at 3 months (125±18 versus 77±13 number of small muscularized vessels/mm² in control, p=0.0014).

### 2.3.6 PlGF-2 does not induce cardiac damage and inflammation

Compared to week 0, 12 and 20 weeks, cTnI level significantly increased at 3 hours after ischemic reperfusion to confirm the myocardial infarction. No difference was found between different treatment groups at 12 and 20 weeks. This proves that PlGF-2 did not induce cardiac necrosis.

Based on the analysis of Sirius red staining, IS was comparable in both groups at 12 and 20 weeks (9.7±5.3 versus 11.5±6.2% PlGF at 12 weeks; 9.6±7.5 versus 12.1±5.1% PlGF at 20 weeks). At 12 and 20 weeks, MAC3⁺ cell density was comparable between the groups in the ischemic and remote regions. However, at 20 weeks MAC3⁺ cell density increased significantly compared to 12 weeks regardless the regions (ischemic or remote non-ischemic) and treatment (PBS or PlGF-2), see Figure 7.

### 2.3.7 PlGF-2 did not promote unstable plaque phenotype:

### Aortic artery stiffness measurement using Doppler pulse wave ultrasound.

As an index of aortic artery stiffness, peak velocities were measured at 5 different positions: in the aortic root, ascending aorta, 3 in aortic arch next to innominate, carotid and subclavian artery (Figure 8). Peak velocity significantly increased 1 month after myocardial infarction in all positions, except in the aortic root. Compared to PBS, application of P1GF-2 did not induce peak velocity difference at 12 or 20 weeks.

### Morphometry, fibrosis, calcification, angiogenesis and inflammation in plaque analysis using histologic stainings.

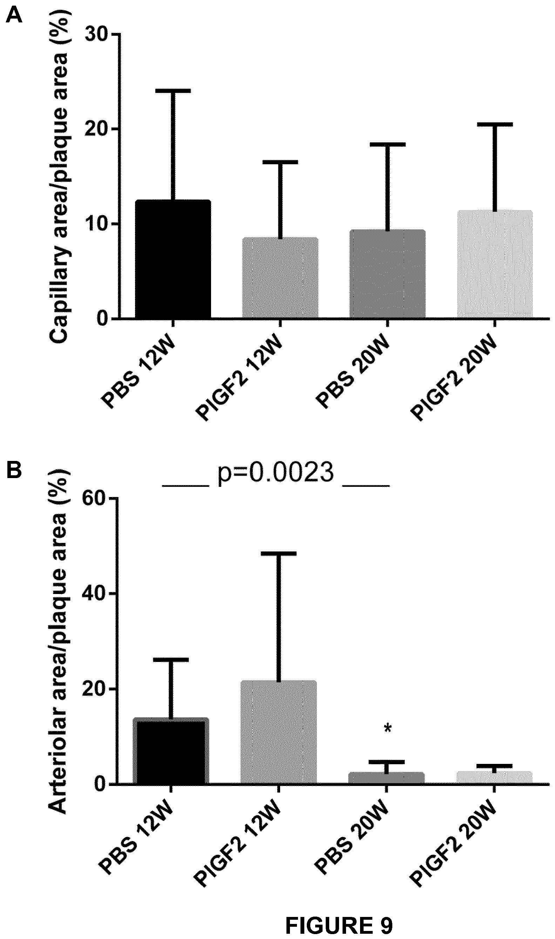

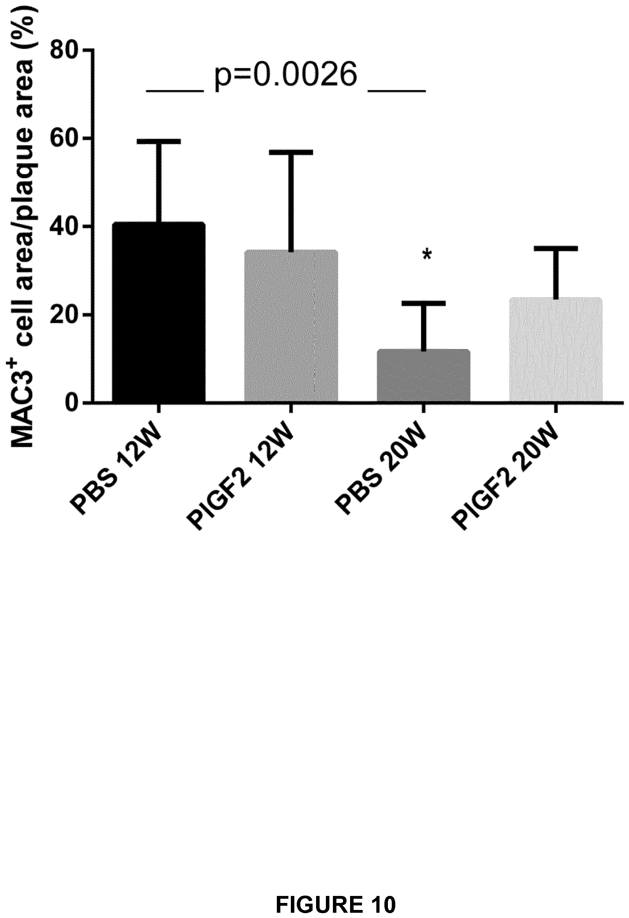

PlGF-2 administration did not increase normalized plaque area, measured with Verhoeff van Gieson staining at 1 month and 3 months after treatment. Nevertheless, the plaque areas became larger at the later stage most likely due to continuous high cholesterol diet feeding. PlGF-2 infusion did not induce any change in the normalized plaque fibrosis (Sirius red staining) and calcification (Alizarin red staining) at 12 and 20 weeks. At 12 weeks, plaque calcification was revealed in 4 mice (of which 2 from the PBS group), while at 20 weeks, in 14 mice (of which 7 from the PBS group). PlGF-2-treatment did not increase the capillary and arteriolar areas in the plaques at 12 or 20 weeks (Figure 9). This represents an additional confirmation that PlGF-2 did not contribute to plaque vulnerability. Compared to PBS, PlGF-2-treated mice showed comparable inflammatory response in the plaque, similar MAC3⁺ cell area over the plaque area at 12 and 20 weeks (figure 10).

Spleen weight and spleen over body weight did not differ between groups at observed time-points.

### 2.3.8 Conclusion

In conclusion, the presented data demonstrate that systemic administration of PlGF-2 significantly limits the progress of HF. More particularly, it is demonstrated herein that PlGF-2 improves neovascularization capacity of the myocardium, contractile function and it preserves cardiac structure without increasing the size of atherosclerotic plaques, without increasing macrophage accumulation and without destabilization of plaques.

Thus the present data support the use of infusion of PlGF-2 protein in patients in the treatment of patients in need of improved myocardial perfusion and function.

The present data further support the use of P1GF-2 for the prevention of cardiac remodeling and to prevent the development of heart failure caused by ischemic cardiomyopathy.

## Claims

1. A composition comprising placental growth factor 2 protein (PIGF-2) for use in treating or preventing a heart failure phenotype in a mammal, wherein said mammal is atherosclerotic.

2. The composition for use according to claim 1 wherein said heart failure phenotype is ventricular dysfunction.

3. The composition for use according to claim 2 wherein said ventricular dysfunction is defined by an increased end-systolic volume.

4. The composition for use according to claim 2 wherein said ventricular dysfunction is defined by a reduced ejection fraction.

5. The composition for use according to claim 2 wherein said ventricular dysfunction is defined by an increased end-systolic volume and by a reduced ejection fraction.

6. The composition for use according to claim 1 wherein said heart failure phenotype is ventricular remodeling.

7. The composition for use according to claim 6 wherein said ventricular remodeling is defined by an increased end-diastolic volume.

8. The composition for use according to claim 2 wherein said ventricular dysfunction is defined by an impaired relaxation, an increased ventricular filling pressure and a preserved ejection fraction.

9. The composition for use according to any of claims 1 to 8 wherein said heart failure phenotype is displayed in the left and/or right ventricle.

10. The composition for use according to any of claims 1 to 9 wherein said PIGF-2 protein is recombinant PIGF-2 protein.

11. The composition for use according to claim 10 wherein said recombinant PIGF-2 protein is obtained from a mammalian cell culture.

12. The composition for use according to claim 11 wherein said mammalian cell culture is a Chinese Hamster Ovary cell culture capable of transiently or constitutively expressing a PIGF-2.

13. The composition for use according to any of claims 1 to 12 wherein said PIGF-2 protein is human PIGF-2 protein.

14. The composition for use according to claim 13 wherein said human PIGF-2 protein is defined by SEQ ID NO:1 or a polymorph thereof.

15. The composition for use according to any of claims 1 to 14 which is for use by systemic administration.

16. The composition for use according to claim 15 wherein said systemic administration is endovascular or subcutaneous administration.

17. The composition for use according to claim 16 wherein said endovascular administration is intravenous administration.

## Patentansprüche

1. Zusammensetzung, umfassend Plazenta-Wachstumsfaktor-2-Protein (PlGF-2) zur Verwendung bei der Behandlung oder Vorbeugung eines Phänotyps des Herzversagens bei einem Säugetier, wobei das Säugetier atherosklerotisch ist.

2. Zusammensetzung zur Verwendung nach Anspruch 1, wobei der Phänotyp des Herzversagens ventrikuläre Dysfunktion ist.

3. Zusammensetzung zur Verwendung nach Anspruch 2, wobei die ventrikuläre Dysfunktion durch ein erhöhtes endsystolisches Volumen definiert ist.

4. Zusammensetzung zur Verwendung nach Anspruch 2, wobei die ventrikuläre Dysfunktion durch eine reduzierte Ejektionsfraktion definiert ist.

5. Zusammensetzung zur Verwendung nach Anspruch 2, wobei die ventrikuläre Dysfunktion durch ein erhöhtes endsystolisches Volumen und durch eine reduzierte Ejektionsfraktion definiert ist.

6. Zusammensetzung zur Verwendung nach Anspruch 1, wobei der Phänotyp des Herzversagens ventrikuläres Remodeling ist.

7. Zusammensetzung zur Verwendung nach Anspruch 6, wobei das ventrikuläre Remodeling durch ein erhöhtes enddiastolisches Volumen definiert ist.

8. Zusammensetzung zur Verwendung nach Anspruch 2, wobei die ventrikuläre Dysfunktion durch eine beeinträchtigte Relaxation, einen erhöhten ventrikulären Fülldruck und eine konservierte Ejektionsfraktion definiert ist.

9. Zusammensetzung zur Verwendung nach einem der Ansprüche 1 bis 8, wobei der Phänotyp des Herzversagens im linken und/oder rechten Ventrikel angezeigt wird.

10. Zusammensetzung zur Verwendung nach einem der Ansprüche 1 bis 9, wobei das PlGF-2-Protein rekombinantes PlGF-2-Protein ist.

11. Zusammensetzung zur Verwendung nach Anspruch 10, wobei das rekombinante PlGF-2-Protein aus einer Säugetierzellkultur erhalten wird.

12. Zusammensetzung zur Verwendung nach Anspruch 11, wobei die Säugetierzellkultur eine Kultur von Ovarienzellen des chinesischen Hamsters ist, die P1GF-2 vorübergehend oder konstitutiv exprimieren kann.

13. Zusammensetzung zur Verwendung nach einem der Ansprüche 1 bis 12, wobei das PlGF-2-Protein menschliches PlGF-2-Protein ist.

14. Zusammensetzung zur Verwendung nach Anspruch 13, wobei das menschliche PlGF-2-Protein durch SEQ ID NO: 1 oder ein Polymorph davon definiert ist.

15. Zusammensetzung zur Verwendung nach einem der Ansprüche 1 bis 14, die zur Verwendung durch systemische Verabreichung vorgesehen ist.

16. Zusammensetzung zur Verwendung nach Anspruch 15, wobei die systemische Verabreichung endovaskuläre oder subkutane Verabreichung ist.

17. Zusammensetzung zur Verwendung nach Anspruch 16, wobei die endovaskuläre Verabreichung intravenöse Verabreichung ist.

## Revendications

1. Composition comprenant la protéine du facteur de croissance placentaire 2 (PlGF-2) pour utilisation dans le traitement prophylactique ou thérapeutique d'un phénotype de défaillance cardiaque chez un mammifère, où ledit mammifère est athérosclérotique.

2. Composition pour utilisation selon la revendication 1, où ledit phénotype de défaillance cardiaque est un dysfonctionnement ventriculaire.

3. Composition pour utilisation selon la revendication 2, où ledit dysfonctionnement ventriculaire est défini par une augmentation du volume systolique final.

4. Composition pour utilisation selon la revendication 2, où ledit dysfonctionnement ventriculaire est défini par une diminution de la fraction d'éjection.

5. Composition pour utilisation selon la revendication 2, où ledit dysfonctionnement ventriculaire est défini par une augmentation du volume systolique final et par une diminution de la fraction d'éjection.

6. Composition pour utilisation selon la revendication 1, où ledit phénotype de défaillance cardiaque est un remodelage du ventricule.

7. Composition pour utilisation selon la revendication 6, où ledit remodelage du ventricule est défini par une augmentation du volume diastolique final.

8. Composition pour utilisation selon la revendication 2, où ledit dysfonctionnement ventriculaire est défini par une anomalie de la relaxation, une augmentation de la pression de remplissage ventriculaire et une conservation de la fraction d'éjection.

9. Composition pour utilisation selon l'une quelconque des revendications 1 à 8, où ledit phénotype de défaillance cardiaque se manifeste dans le ventricule gauche et/ou droit.

10. Composition pour utilisation selon l'une quelconque des revendications 1 à 9, où ladite protéine PlGF-2 est une protéine PlGF-2 recombinante.

11. Composition pour utilisation selon la revendication 10, où ladite protéine PlGF-2 recombinante est obtenue à partir d'une culture de cellule mammalienne.

12. Composition pour utilisation selon la revendication 11, où ladite culture de cellules mammaliennes est une culture de cellules d'ovaire de hamster chinois capables d'exprimer de façon transitoire ou constitutive un PlGF-2.

13. Composition pour utilisation selon l'une quelconque des revendications 1 à 12, où ladite protéine PlGF-2 est une protéine PlGF-2 humaine.

14. Composition pour utilisation selon la revendication 13, où ladite protéine PlGF-2 humaine est définie par la SEQ ID NO:1 ou l'un de ses polymorphes.

15. Composition pour utilisation selon l'une quelconque des revendications 1 à 14, qui est destinée à une utilisation par administration systémique.

16. Composition pour utilisation selon la revendication 15, où ladite administration systémique est une administration endovasculaire ou sous-cutanée.

17. Composition pour utilisation selon la revendication 16, où ladite administration endovasculaire est une administration intraveineuse.
